**Europäisches Patentamt**

**European Patent Office**

**Office européen des brevets**

(11) Veröffentlichungsnummer: **0 187 653 B1**

(12) **EUROPÄISCHE PATENTSCHRIFT**

(45) Veröffentlichungstag der Patentschrift: **13.05.92**

(21) Anmeldenummer: **86100105.5**

(22) Anmeldetag: **07.01.86**

(51) Int. Cl.⁵: **A61K 31/735**, A61K 31/715

(54) **Galleersetzende, die Fettverdauung fördernde Arzneimittelpräparate und Verfahren zu ihrer Herstellung.**

(30) Priorität: **10.01.85 HU 7585**

(43) Veröffentlichungstag der Anmeldung:
**16.07.86 Patentblatt 86/29**

(45) Bekanntmachung des Hinweises auf die
Patenterteilung:
**13.05.92 Patentblatt 92/20**

(84) Benannte Vertragsstaaten:
**AT BE CH DE FR GB IT LI NL SE**

(56) Entgegenhaltungen:
**AT-B- 374 684**
**GB-A- 2 092 890**
**US-A- 4 371 673**

(73) Patentinhaber: **CHINOIN Gyogyszer és Vegyészeti Termékek Gyára RT.**
**To utca 1-5**
**H-1045 Budapest IV(HU)**

(72) Erfinder: **Szejtli, J zsef, Dr.**
**Endrödi S. u. 38/40**
**H-1026 Budapest(HU)**
Erfinder: **Szente, Lajos, Dr.**
**Harrer Pál u. 20**
**H-1033 Budapest(HU)**
Erfinder: **Káloy, Katalin, Dr.**
**Ibrahim u. 15 ii. 7.**
**H-1113 Budapest(HU)**
Erfinder: **Marton, Jenö, Dr.**
**Szél u. 19**
**H-1035 Budapest(HU)**
Erfinder: **Gerloczy, Andrea, Dr.**
**Tizedes u. 5**
**H-1025 Budapest(HU)**

(74) Vertreter: **Bartsch, Elisabeth, Dr.**
**Patentanwälte Lotterhos & Partner Lichtensteinstrasse 3**
**W-6000 Frankfurt am Main 1(DE)**

**Beschreibung**

Die Erfindung betrifft Arzneimittelpräparate, die die Gallenflüssigkeit ersetzen und die Verdauung und Resorption von Fetten fördern. Die Arzneimittelpräparate enthalten erfindungsgemäß als Ersatz der natürlichen Galle methyliertes $\beta$-Cyclodextrin. Die Erfindung betrifft auch ein Verfahren zur Herstellung dieser Arzneimittelpräparate.

Die Galle ist ein von den Leberzellen abgesondertes Sekret, das entweder unmittelbar ins Duodenum gelangt oder vorübergehend in der Gallenblase gespeichert wird, wo es zum Teil konzentriert und den Bedürfnissen der Verdauung entsprechend an den Darm weitergegeben wird. Die unmittelbar entleerte sog. Lebergalle ist immer dünnflüssig, die aus der Blase entleerte sog. Blasengalle ist meistens wesentlich dicker, die Konzentration wird häufig mehr als verzehnfacht.

Die spezifisch die meisten Kalorien enthaltende Komponente der menschlichen Ernährung wird von den Lipiden, vor allem den Triglyceriden gebildet. Etwa 40 % des Gesamtkaloriengehaltes normaler menschlicher Nahrung stammen aus dem Verzehr von täglich etwa 100 g Lipiden. Im gesunden Zustand werden höchstens 5 % der aufgenommenen Fette unverdaut mit dem Stuhl entleert. Die Triglyceride sind in Wasser unlöslich, ihr Abbau zu hauptsächlich Fettsäuren und Monogylceriden geht im oberen Abschnitt des Dünndarms vor sich und wird von der Pankreaslipase katalysiert. Bedingung für die optimale Lipaseaktivität ist die Gegenwart von Gallensäuren. Durch die solubilisierende Wirkung der Gallensäuren werden die Lipide zu feinen Teilchen dispergiert. Die Gegenwart von Gallensäuren ist nicht nur für den enzymatischen Abbau, sondern auch für die Absorption der wasserunlöslichen Produkte der Lipolyse erforderlich. Die natürliche Galle enthält Cholsäure, Desoxycholsäure und in sehr geringen Mengen noch zwei weitere Gallensäurederivate, die alle in unterschiedlichem Maße hydroxylierte Abkömmlinge der Cholansäure sind. In der Galle bilden die Gallensäuren, an Glykokoll oder Taurin gebunden, Glykocholsäure oder Taurocholsäure und liegen in Form ihrer wasserlöslichen Natriumsalze vor. Die funktionellen Teile der in den Darm gelangenden Galle werden von den Gallensäuren gebildet, die zu 90 % aus dem Blut resorbiert werden und rezirkulieren. Im Harn erscheint Gallensäure nur, wenn die Leberfunktion geschädigt ist. Im Organismus werden die Gallensäuren nicht abgebaut. Sie sind toxisch und verursachen Hämolyse, wenn sie in den Blutkreislauf gelangen.

Die Gründe für unzureichende Lipidverdauung (Lipidmalabsorption) können folgende sein: verringerte Synthese der Gallensäuren, Hemmung ihres Eintrittes in den Darm, Verminderung der wirksamen Konzentration an konjugierten Gallensäuren, erhöhter Verlust an Gallensäuren. Durch unzureichende Lipidverdauung treten folgende Symptome auf: Blähungen, Darmkrämpfe, häufige abnormale Stuhlentleerung, Diarrhoe, Kalorienverlust, Gewichtsverlust und als Folge unzureichender Absorption der fettlöslichen Vitamine treten, auf Hypovitaminose hinweisende Symptome auf.

Bei unzureichender Lipidverdauung muß die natürliche Galle ersetzt werden. In der gegenwärtig angewendeten Therapie wird die Galle durch Gallensäurensalze oder durch Detergentien ersetzt. Gallensäurensalze sind zum Beispiel in den Suprachol®-Dragees (Dehydrocholsäure), Bilagit®-Dragees (Cholsäurenatriumsalz), Bilival®-Tabletten (mit Lecithin gebildeter Komplex des Cholsäurenatriumsalzes) enthalten. Als Detergentien werden zum Beispiel Tween 60®, Polysorbat 80® usw. verwendet, d.h. die Gemische der Stearinsäure- und Ölsäureester von Polyalkoholen.

Die Anwendung von Detergentien hat den Nachteil, daß sie in verhältnismäßig hohen Dosen (täglich 6 g) eingesetzt werden müssen, weil sie mit den Lipiden nur Micellen, jedoch keine hochstabilen Komplexe bilden. Deshalb können sie nur in größeren Mengen eine geeignete Wirkung ausüben.

Durch die Behandlung mit gallensäurenhaltigen Arzneimitteln wird die Galleproduktion angeregt. Werden die Gallensäuren als Ersatz für die natürliche Galle in Fällen verabreicht, in denen keine Galle in den Darm fließt (z.Beispiel Gallenfistel), so kann die gehemmte Fettresorption durch die oral gegebenen Gallensäuren verbessert werden. Wenn jedoch der Eintritt der Galle in den Darm gehemmt ist (Gelbsucht, Gallensteinkolik), so dürfen keine gallensäurenhaltigen Mittel verabreicht werden. Auch bei an Hepatitis Leidenden dürfen keine Gallensäuren gegeben werden, durch die im Organismus rezirkulierten Gallensäuren würde nur die ohnehin in ihrer Funktion geschädigte Leber belastet. Auch durch den engen Zusammenhang, der zwischen dem Cholesterinspiegel des Blutes und der Gabe von Gallensäuren besteht, ist eine Verbesserung der Fettverdauung durch Verabreichung von Gallensäuren kontraindiziert.

Die Verdauung und Resorption von Fetten fördernde Arzneimittel sind demnach erforderlich, wenn die Funktion der Galle nicht völlig normal ist, andererseits ist jedoch die Verabreichung von Gallensäuren als Ersatz der natürlichen Galle in vielen Fällen mit Nachteilen verbunden.

Ziel der Erfindung war es, als Ersatz für die natürliche Galle ein Arzneimittelpräparat zu schaffen, welches auf einem von den bisherigen Arzneimitteln völlig abweichenden Prinzip beruht und keine Verbindungen enthält, die von der Leber ausgeschieden und in der Gallenblase gespeichert werden.

Überraschenderweise wurde nun gefunden, daß für diesen Zweck die methylierten Cyclodextrine geeignet sind.

Wird Stärke mit eines speziellen stärkeabbauenden Enzym, der Cyclodextrin-transglykosidase, abgebaut, so entstehen Dextrine mit cyclischer Struktur, und zwar die sich aus 6, 7 beziehungsweise 8 Glucoseeinheiten aufbauenden $\alpha$-, $\beta$- beziehungsweise $\gamma$-Cyclodextrine. Für sie ist charakteristisch, daß sie wasserlöslich sind und mit unterschiedlichen apolaren sog. Gastmolekülen geeigneter Größe Einschlußkomplexe bilden. Die Wasserlöslichkeit der Einschlußkomplexe ist gering.

Einschlußkomplexe von methylierten Cyclodextrinen mit Retinoiden sind aus der US-A-4 371 673 bekannt.

Eigene frühere Versuche haben erseben, daß die Wasserlöslichkeit, verglichen mit den unsubstituierten Cyclodextrinen, bedeutend steigt, wenn man aus den Cyclodextrinen partiell methylierte Derivate bildet. Auch die Wasserlöslichkeit der mit methylierten Cyclodextrinen gebildeten Einschlußkomplexe ist wesentlich größer als derjenigen die mit unsubstuierten Cyclodextrinen gebildet worden sind. Durch derartige Komplexbildung mit methylierten Cyclodextrinen können aus nicht oder kaum wasserlöslichen biologisch aktiven organischen Verbindungen sogar injizierbare Lösungen hergestellt werden (vgl. AT-B-374 684).

Von allen methylierten Cyclodextrin-Derivaten scheint für die praktische Anwendung das Heptakis-(2,6-di-0-methyl)-$\beta$-cyclodextrin am geeignetsten, weil die Wasserlöslichkeit der in geringerem oder höherem Maße partiell methylierten Cyclodextrine geringer ist. Das Heptakis(2,6-di-0-methyl)-$\beta$-cyclodextrin, kurz Dimethyl-$\beta$-cyclodextrin (im folgenden DIMEB) ist ein in kaltem Wasser sehr gut lösliches weißes Pulver, das die besondere Eigenschaft hat, sich bei Erwärmen der Lösung bei einer von der Konzentration abhängenden Temperatur (45-60 °C) plötzlich kristallin auszuscheiden und bei Abkühlen wieder in Lösung zu gehen. Das Dimethyl-$\beta$-cyclodextrin wird durch partielle Methylierung des $\beta$-Cyclodextrins hergestellt. Dafür sind verschiedene Verfahren bekannt (ungarische Patentschrift Nr. 180 580 beziehungsweise veröffentlichte ungarische Patentanmeldung Nr. 838/83). Die Substanz ist auch in organischen Lösungsmitteln löslich, sie wird, wie Tierversuche gezeigt haben, bei oraler Verabreichung nicht resorbiert, an Mäusen konnte bis zu einer Dosis von 3000 mg/kg keine Toxizität festgestellt werden. Bei intravenöser Verabreichung wird die gesamte Substanz innerhalb von 6 Stunden durch die Nieren ausgeschieden.

Überraschenderweise wurde gefunden, daß DIMEB den Fettgehalt der Nahrung durch Bildung von Einschlußkomplexen dispergiert, dadurch für den Abbau durch Lipase zugänglich macht und auf diese Weise Verdauung und Resorption der Fette fördert, d.h. als Ersatz für die natürliche Gelle geeignet ist. Die galleersetzende Wirkung vor DIMEB konnte durch Versuche sowohl in vitro wie auch in vivo nachgewiesen werden.

Unter in vitro-Bedingungen wurde die mit Lipase vorgenommene Hydrolyse von Triglyceriden untersucht, und es wurde beobachtet, daß die Wirkung der Lipase in Lösungen durch DIMEB gesteigert wird.

Unter in vivo-Bedingungen wurde an unterschiedlichen Testtieren (Ratten, Kaninchen usw.) die Rolle von DIMEB in der Verdauung beziehungsweise der Resorption von Fetten untersucht. Bei den Versuchen wurde durch einen operativen Eingriff verhindert, daß die Tiere ihre eigene Galle verwerten können. Zusammen mit den Triglyceriden wurde dann oral auch DIMEB appliziert. Es wurde beobachtet, daß sich durch die Wirkung von DIMEB Fettverdauung und -resorption normalisierten.

Die Erfindung betrifft partiell methyliertes $\beta$-Cyclodextrin, vorzugsweise DIMEB, zur Anwendung als galleersetzende, die Verdauung und Resorption der Fette fördernde Wirkstoffe sowie Arzneimittelpräparate, die diese Wirkstoffe: partiell methyliertes $\beta$-Cyclodextrin, vorzugsweise DIMEB, zusammen mit den in der Arzneimittelherstellung üblichen Träger- und/oder Hilfsstoffen enthalten. Die galleersetzenden und die Fettverdauung fördernden Wirkstoffe und die üblichen Träger- und/oder Hilfsstoffe liegen in den Arzneimitteln im Verhältnis 5 - 95 % Wirkstoff : 95 - 5 % Träger- und/oder Hilfsmittel vor. Die Herstellung erfolgt in an sich bekannter Weise.

Da die durch unzureichende Gallenfunktion verursachte mangelhafte Fettverdauung mit schmerzhaften Darmkrämpfen verbundene mikrobielle Zersetzungsprozesse zur Folge hat, enthalten die Arzneimittelpräparate zweckmäßig auch desinfizierend, krampflösend und auf die Darmperistaltik stimulierend wirkende Komponenten, beziehungsweise Verdauungsenzyme.

In den folgenden Beispielen werden die Herstellung der erfindungsgemäßen Arzneimittelpräparate sowie die in vitro und in vivo vorgenommenen Versuche zur Untersuchung der galleersetzenden Wirkung von DIMEB näher beschrieben; die Erfindung ist jedoch nicht auf die Beispiele beschrankt.

Beispiele für Arzneimittelpräparate

Beispiel 1

| Heptakis-(2,6-di-0-methyl)-$\beta$-cyclodextrin | 250 mg |
| Talcum | 50 mg |
| Polyvidonum® | 10 mg |
| Magnesium stearinicum | 4 mg |
| Lactosum | 36 mg |
| | 350 mg |

Der Wirkstoff und die Hilfsstoffe werden miteinander homogenisiert und mit der alkoholischen Lösung des Polyvidons granuliert. Nach dem Trocknen des Granulates wird es in an sich bekannter Weise zu Tabletten von 350 mg Gewicht verpreßt.

Beispiel 2

| Heptakis-(2,6-di-0-methyl)-$\beta$-cyclodextrin | 50 mg |
| Lactosum | 25 mg |
| Talcum | 19 mg |
| Magnesium stearinicum | 2 mg |
| Polyvidonum® | 4 mg |
| | 100 mg |

Die Tabletten werden auf die im Beispiel 1 beschriebene Weise hergestellt mit dem Unterschied, daß Tabletten zu je 100 mg Gewicht gepreßt werden.

Beispiel 3

| Heptakisis-(2,6-di-0-methyl)-$\beta$-cyclodextrin | 200 mg |
| Papaverin hydrochloricum | 20 mg |
| Natrium choleinicum | 50 mg |
| Talcum | 14 mg |
| Lactosum | 10 mg |
| Magnesium stearinicum | 4 mg |
| Polyvidonum® | 2 mg |
| | 300 mg |

Die Drageekerne werden auf die im Beispiel 1 beschriebene Weise bereitet und dann in an sich bekannter Weise in einem Überdruckgerät mit einem Film dragiert.

Beispiel 4

| Heptakis-(2,6-di-0-methyl)-$\beta$-cyclodextrin | 150 mg |
| Polyoxäthylen-sorbitan-monostearat (Tween 60) | 150 mg |
| Aerosil 972® | 12 mg |
| Magnesium stearinicum | 4 mg |
| Talcum | 30 mg |
| Polyvidonum® (PVP) | 4 mg |
| | 350 mg |

Das Tween 60® wird mit dem Aerosil® in kleinen Portionen homogenisiert, Wirk- und Hilfsstoffe werden zugemischt, und das Gemisch wird mit der alkoholischen Lösung des PVP granuliert und nach dem Trocknen zu Tabletten verpreßt.

Beispiele zur Wirkung

Beispiel 5

Wirkungssteigerung der Lipase in Dimethyl-$\beta$-cyclodextrin-Lösungen

Triglyceride werden in wäßrigem Medium von Lipase nur mit sehr geringer Geschwindigkeit gespalten. Entweder muß ein organisches Lösungsmittel zugesetzt werden, das die Löslichkeit der Triglyceride verbessert, gleichzeitig aber die Enzymaktivität verrringert, oder es muß ein Detergens, zweckmäßig Galle, verwendet werden. Die Wirksamkeit von Galle wurde mit der von Dimethyl-$\beta$-cyclodextrin bei 37 °C verglichen, indem den Reaktionsgemischen zu unterschiedlichen Zeitpunkten Proben entnommen wurden und deren Gehalt an gebildeten freien Fettsäuren mit Natriumhydroxyd titriert wurde.

In einem Volumen von 1,5 ml befanden sich 4 $\mu$Mol Substrat (Triolein oder Olivenöl), 10 mg Rinderserumalbumin, 75 $\mu$Mol NaCl, 50 $\mu$Mol Phosphatpuffer (pH = 7,4) und 0,25 ml Enzymlösung. Als Enzyme fanden Lipase (Hersteller: Serva) als Lösung von 1 mg in 10 ml beziehungsweise 0,5 g Pankreaspulver, suspendiert in 10 ml destilliertem Wasser (nach Filtrieren als schwach opalisierende Lösung) Verwendung.

Die zu untersuchenden Ansätze wurden folgendermaßen zusammengestellt:

0,5 ml Puffer + 10 mg Serumalbumin + 3,54 mg Substrat + 0,25 ml Enzymlösung, dazu

+ a) 0,25 ml verdünnte Galle + 0,50 ml dest. Wasser oder

+ b) 50 mg DIMEB in 0,75 ml Wasser gelöst, oder

+ c) 0,75 ml Wasser

Wie aus der Tabelle 1 hervorgeht, wird die Wirkung der Lipase durch Dimethyl-$\beta$-cyclodextrin bedeutend beschleunigt.

Tabelle 1

Triglycerid-Hydrolyse in vitro in Gegenwart von Galle bzw. DIMEB

| Reaktions- zeit, h | verbrauchte Menge an 0,005 n NaOH, ml | | |
|---|---|---|---|
| | Galle I: 5fach verdünnte Schweinegalle, Substrat: Olivenöl, Enzym: Pakreassuspension | | |
| | a) Galle | b) DIMEB | c) Kontrolle |
| 0,5 | 0,82 | 1,74 | 0,0 |
| 1 | 1,17 | 2,00 | 0,36 |
| 2 | 1,37 | 2,20 | 0,48 |
| 19 | 2,62 | 4,77 | 0,63 |
| Anstieg der Reaktionsgeschwindigkeit | 4 x | 7,4 x | |
| | Galle II: 10fach verdünnte Hühnergalle, Substrat: Glycerintrioleat, Enzym: Lipase | | |
| | a) Galle | b) DIMEB | c) Kontrolle |
| 1 | 0,20 | 0,34 | 0,0 |
| 2 | 0,32 | 0,80 | 0 |
| 3 | 0,48 | 1,16 | 0 |
| 21 | 0,58 | 1,86 | 0,1 |
| Anstieg der Reaktionsgeschwindigkeit | 6 x | 18 x | |
| | Galle III: 5fach verdünnte Hühnergalle, Substrat: Glycerintrioleat, Enzym: Lipase | | |
| | a) Galle | b) DIMEB | c) Kontrolle |
| 1 | 0,26 | 0,28 | 0 |
| 2 | 0,46 | 0,68 | 0 |
| 3 | 0,74 | 1,20 | 0,14 |
| Anstieg der Reaktionsgeschwindigkeit | 5 x | 8,5 x | |

Beispiel 6

Verstärkung der Lipidabsorption durch orale Verabreichung von DIMEB (Ratte)

Die Wirkung von DIMEB auf die Resorption von Speiseöl wurde an intakten Ratten untersucht. Ziel des Versuches war es zu klären, inwiefern DIMEB das Emulgieren und die Resorption von Speiseöl beeinflußt, wenn die Gallenfunktion nicht beeinträchtigt ist. Den Versuchstieren wurden per os (Magensonde) 2 ml Sonnenblumenöl, beziehungsweise 2 ml Sonnenblumenöl und 50 mg DIMEB appliziert. Der Gehalt des Blutplasmas an Triglycerid und freien Fettsäuren wurde zu unterschiedlichen Zeitpunkten bestimmt (Tabellen 1 und 2).

In der ersten Stunde nach der Behandlung besteht zwischen dem Triglyceridspiegel der Kontrolle und der behandelten Gruppen kein signifikanter Unterschied (Tabelle 2). In der 4. Stunde ist der Unterschied zwischen der Kontrolle und der mit Öl behandelten Gruppe noch nicht signifikant, was anzeigt, daß der Verdauungsprozeß noch andauert, Bei der mit Öl und DIMEB behandelten Gruppe ist jedoch,verglichen mit den beiden anderen Gruppen,bereits eine signifikant höhere Triglycerid-Konzentration im Plasma zu verzeichnen. In der 4. Stunde ist die Triglycerid-Konzentration bei der mit DIMEB behandelten Gruppe um mehr als 100 % höher. In der 6. Stunde steigt auch der Triglyceridspiegel der nur mit Öl behandelten Gruppe im Vergleich zur Kontrolle signifikant an, bleibt jedoch immer noch signifikant unter dem Wert der mit Öl und DIMEB behandelten Gruppe. Eine ähnliche Tendenz ist auch in Hinblick auf die freien Fettsäuren zu verzeichnen (Tabelle 3).

Die Triglycerid-Konzentration des Plasmas wurde nach Van Handel (Van Hanel E. 1961 Clin. Chem. 7, 249-251), die Konzentration der freien Fettsäuren nach Dumcombe (Dumcombe W.G., 1963, Biochem. J. 88, 7-10) bestimmt.

Tabelle 2

| Triglycerid-Konzentration im Plasma normaler Ratten (ausgedrückt in mg/100 ml), Behandlung: 2 ml Speiseöl beziehungsweise 2 ml Speiseöl + 50 mg DIMEB | | | |
|---|---|---|---|
| Zeit, h | Kontrolle | Öl | Öl + DIMEB |
| 0 | 49 ± 12 | | |
| 1 | | 43 ± 8 | 44 ± 6 |
| 2,5 | | 72 ± 18 | 61 ± 18 |
| 4 | | 58 ± 11 | 121 ± 7 |
| 6 | | 88 ± 5 | 122 ± 15 |

Tabelle 3

| Konzentration freier Fettsäuren im Plasma normaler Ratten (ausgedrückt in mg/100 ml), Behandlung: 2 ml Speiseöl beziehungsweise 2 ml Speiseöl + 50 mg DIMEB | | | |
|---|---|---|---|
| Zeit | Kontrolle | Öl | Öl + DIMEB |
| 0 | 97 ± 36 | | |
| 1 | | 240 ± 70 | 326 ± 15 |
| 2,5 | | 412 ± 146 | 494 ± 183 |
| 4 | | 310 ± 93 | 641 ± 125 |
| 6 | | 412 ± 88 | 347 ± 65 |

Die Versuchsergebnisse beweisen, daß DIMEB in gesunden, intakten Tieren die Resorption von Triglyceriden wirkungsvoll verstärkt.

Beispiel 7

Ersatz der Gelle (Kaninchen)

Durch Abbinden der Gallenleitung (Choledochus-Ligstur) der Versuchstiere wurde verhindert, daß diese zur Verdauung der Lipide ihre eigene Galle verwerten können. Bei normaler Pankreasabtrennung wurde nach oraler Verabreichung von Lipiden die Triglycerid-Konzentration in Blutplasme untersucht. Die Versuchstiere (Orychtolagus cuniculus) erhielten vor dem Versuch 10 Tage lang Standardnahrung, deren organische Bestandteile folgende Zusammensetzung hatten:

| | |
|---|---|
| Roheiweiß | 16,0 % |
| lösliche Kohlehydrate | 42,7 % |
| Rohfasern | 12,6 % |
| Rohfett | 7,3 % |
| Vitaminmischung | 1,0 %. |

100 g Nahrung enthielten 1360 Joule. Vor dem operativen Eingriff bekamen die Tiere 12-24 Stunden lang kein Futter, jedoch Wasser ad libitum. Innerhalb einer Versuchsreihe wurden 2500-3000 g schwere Tiere des gleichen Geschlechtes verwendet, die Operationen wurden gleichzeitig vorgenommen. Als Kontrolle dienten Tiere, die scheinoperiert waren. Der Versuch begann 48 Stunden nach der Operation. Die Untersuchungssubstanzen wurden per os mittels einer Magensonde verabreicht, Blut wurde aus der Vena auricularis marginalis entnommen.

In der ersten Versuchsreihe wurden der ersten Tiergruppe 3 ml Sonnenblumenöl, der zweiten Gruppe 3 ml Sonnenblumenöl zusammen mit in 3 ml Wasser gelösten 200 mg DIMEB appliziert, die dritte Gruppe, die Kontrolle (Scheinoperation) erhielt 6 ml Wasser.

Gemäß den Daten der Tabelle 4 verläuft in Abwesenheit von Gallensäuren und Anwesenheit von Lipase die Verdauung der Lipide sehr langsam, denn die Konzentration der Trgiglyceride im Plasma stieg innerhalb der sechsstündigen Versuchsperiode kaum an. Bei der Gruppe, die zusammen mit dem Speiseöl auch DIMEB erhalten hatte, war bereits von der ersten Stunde an ein augenfälliges, signifikantes Ansteigen des Triglyceridspiegels zu beobachten. In der 6. Stunde war die Triglycerid-Konzentration bei der mit Speiseöl und DIMEB behandelten Gruppe fast dreimal so hoch wie bei der nur mit Speiseöl behandelten Gruppe.

Es ist bekannt, daß die Verdauung von Fetten langsamer verläuft als die von Ölen. Deshalb wurde in der zweiten Versuchsreihe die Veränderung der Triglycerid-Konzentration im Plasma der Versuchstiere nach der oralen Verabreichung von 5 ml Schweinefett beziehungsweise 5 ml Schweinefett + 300 mg

Tabelle 4

| Die Wirkung von nach einer Choledochus-Ligatur vorgenommenen Behandlung auf die Triglycerid-Konzentration im Plasma von Kaninchen (Konz. in mg/100 ml), Behandlung: 3 ml Sonnenblumenöl beziehungsweise 3 ml Sonnenblumenöl + 200 mg DIMEB; Kontrolle: 6 ml Wasser | | | |
|---|---|---|---|
| absolute Kontrolle: 51,0 ± 13,82 mg/100 ml | | | |
| operierte Kontrolle, Stunde Null: 49,03 ± 16,57 mg/100 ml | | | |
| Zeit, h | Kontrolle | Öl | Öl + DIMEB |
| 0 | 36 ± 7 | 46 ± 5 | 52 ± 19 |
| 1 | 34 ± 5 | 57 ± 10 | 96 ± 32 |
| 2,5 | 30 ± 5 | 47 ± 10 | 87 ± 30 |
| 4 | 46 ± 10 | 40 ± 6 | 61 ± 14 |
| 6 | 70 ± 6 | 45 ± 10 | 125 ± 3 |

Tabelle 5

| Die Wirkung von nach einer Choledochus-Ligatur vorgenommenen Behandlung auf die Triglycerid-Konzentration im Plasma von Kaninchen (Konz. in mg/100 ml), Behandlung: 5 ml Schweinefett beziehungsweise 5 ml Schweinefett + 300 mg DIMEB; Kontrolle: 6 ml Wasser | | | |
|---|---|---|---|
| Zeit, h | Kontrolle | Fett | Fest + DIMEB |
| 0 | 49 ± 14 | 46 ± 5 | 58 ± 19 |
| 1 | 34 ± 5 | 57 ± 10 | 104 ± 30 |
| 2,5 | 30 ± 5 | 47 ± 10 | 92 ± 26 |
| 4 | 46 ± 10 | 40 ± 6 | 83 ± 25 |
| 6 | 70 ± 6 | 45 ± 10 | 125 ± 3 |

DIMEB (Kontrolle: 6 ml Wasser) gemessen. Die Ergebnisse zeigt Tabelle 5. Bei den mit Fett und DIMEB behandelten Tieren stieg die Triglycerid-Konzentration, verglichen mit der anderen Gruppe, schon nach einer Stunde um etwa 100 % an. Diese Tendenz hielt über die gesamte Dauer des sechsstündigen Versuches permanent an. Zu jeden Zeitpunkt war die Konzentration signifikant höher als bei den anderen beiden Gruppen.

Die beiden Versuchsreihen beweisen eindeutig, daß bei einem experimentell hervorgerufenen akuten Gallenmangel DIMEB die Dispergierung der Lipide, die Verdauungsfunktion der Lipase uni die Absorption der entstehenden Fettsäuren signifikant fördert, d.h. fähig ist, die natürliche Galle zu ersetzen.

Beispiel 8

Ersatz der Galle (Ratten)

In der ersten Versuchsreihe wurde die Gesamtkonzentration an Lipiden im Plasma von Ratten mit Gallenmangel untersucht. Die Versuchstiere (Etymis rattus var. albino) erhielten vor den Experiment 10 Tage lang Standardfutter folgender Zusammensetzung:

| | |
|---|---|
| Roheiweiß | 19,6 % |
| Rohfasern | 5,6 % |
| lösliche Kohlehydrate | 49,2 % |
| Fett | 4,6 % |
| Vitaminmischung | 1,0 % |

100 g Futter hatten einen Energiegehalt von 1380 Joule.

Die Choledochus-Ligatur wurde unter Äthernarkose vorgenommen. 48 Stunden nach der Operation wurden oral 1,5 ml Schweinefett beziehungsweise 1,5 ml Schweinefett + 75 mg DIMEB verabreicht. Nach 4,5 Stunden wurden die Tiere dekapitiert, die Totallipidkonzentration des Plasmas wurde bestimmt (nach de La Huerga et al.: Amer. J. Clin. Pathol. 23, 1163 [1953]). Wie die Daten der Tabelle 6 zeigen, änderte sich die Totallipidkonzentration nach der Ligatur nicht wesentlich. Bei den Tieren, die nur mit Fett behandelt und einer Gallenleiter-Ligatur unterzogen worden waren, war-verglichen mit der Versuchsgruppe und der Kontrolle-ein signifikanter Anstieg der Totallipidkonzentration zu verzeichnen. Ein bedeutender, signifikanter Anstieg der Totallipidkonzentration gegenüber allen drei anderen Gruppen war bei den mit Fett und DIMEB behandelten Tieren zu beobachten. Während im Falle der nur mit Fett behandelten Tiere die Totallipidfraktion im Blutplasma auf das 2,5fache anstieg, betrug dieser Wert bei den mit Fett und DIMEB behandelten das 5fache.

In einer anderen Versuchsreihe wurde der Totalllpidgehalt der von an Gallenmangel leidenden Ratten entleerten Faeces untersucht. 72 Stunden nach Vornahme der Ligatur wurde die orale Behandlung mit täglich 1 ml Schweinefett beziehungsweise 1 ml Schweinefett und 150 mg DIMEB begonnen und über 5 Tage fortgeführt. Die Faeces wurden in den ersten 4 Tagen der Behandlung gesammelt. Vier Stunden nach der am 5. Tag vorgenommenen Behandlung wurden die Tiere dekapitiert, die ihnen entnommenen Proben wurden aufgearbeitet. In der Tabelle 7 sind die Veränderungen der Lipidmengen in den 4 Tage lang ausseschiedenen Faeces zusammengefaßt. Verglichen mit den intakten und normal ernährten Tieren war bei den ebenfalls normal ernährten, aber mit einer Gallenleiterligatur versehenen Tieren die mit den Faeces entleerte Lipidamenge signifikant höher. Die mit Fett gefütterten Tiere entleerten außerordentlich viel Lipid. Durch die zusätzliche Gabe von täglich 150 mg DIMEB wurde die Lipidentleerung jedoch drastisch gesenkt. Durch diese Behandlung wurde das eingebrachte Fett zu beinahe 100 % absorbiert.

In völliger Übereinstimmung mit diesen Ergebnissen stehen Totalllpid- und Triglycerid-Konzentration im Plasma und die Triglycerid-Konzentration in der Leber (Tabelle 7). Die Totallipidkonzentraton im Plasma ist bei den mit Fett und bei den mit Fett und DIMEB behandelten Tieren signifikant höher als bei den mit einer Ligatur versehenen Tieren und der Kontrolle. Wesentlich ist jedoch, daß bei den mit Fett und DIMEB behandelten Tieren der Wert signifikant höher ist als bei den nur mit Fett behandelten.

Völlig ähnlich ist die Tendenz im Falle der Triglycerid-Konzentration des Plasmas. Zwischen den nur mit Fett und den mit Fett und DIMEB behandelten Tieren besteht signifikante Abweichung. Bei den mit einer Ligatur versehenen, ansonsten nicht behandelten Tieren war die Triglyceridkonzentration der Leber signifikant gering. Die Behandlung dieser Gruppe mit Fett hat einen -Anstieg der Triglycerid-Konzentration auf den Wert der Kontrolle zur Folge. Die Behandlung mit Fett und DIMEB verursacht, bezogen auf die vorherige Gruppe, einen signifikanten Anstieg der Triglycerid-Konzentration.

Zum Nachweis dessen, daß der Anstieg des Lipidspiegels im Blut nicht die Folge einer Mobilisierung des Lipidgehaltes von Organen (Leber, Fettgewebe) ist, sondern aus der Steigerung der Fettabsorption stammt, wurden bei normalem Futter gehaltene Ratten 31 Tage lang oral mit täglich 150 beziehungsweise 300 mg/kg DIMEB behandelt. Wie die Tabelle 9 zeigt, hatte das DIMEB weder auf den Triglyceridgehalt des Plasmas noch auf den der Leber einen Einfluß.

Tabelle 6

| Die Wirkung von nach einer Choledochus-Ligatur vorgenommenen Behandlungen auf die Totallipidkonzentration in Blutplasma von Ratten (Konz. in mg/100 ml), Behandlung: 1,5 ml Fett beziehungsweise 1,5 ml Fett + 75 mg DIMEB | | |
| --- | --- | --- |
| Kontrolle | vor der Ligatur<br>nach Ligatur | 207,1 ± 43,3<br>266,6 ± 39,5 |
| behandelt | mit Fett<br>Fett + DIMEB | 657,7 ± 262,6<br>1266,6 ± 86,5 |

Tabelle 7

| Fettentleerung in Kot von an Gallenmangel leidenden Ratten (in g/100g), Behandlung: 1 ml Fett bzw. 1 ml Fett + 150 mg DIMEB täglich, über 5 Tage | | |
|---|---|---|
| Kontrolle | vor der Ligatur | $0,44 \pm 0,23$ |
|  | nach Ligatur | $4,08 \pm 1,96$ |
| behandelt | mit Fett | $27,55 \pm 4,72$ |
|  | Fett + DIMEB | $6,16 \pm 2,37$ |

Tabelle 8

Totallipid- und Triglyceridgehalt im Plasma sowie Triglyceridgehalt in der Leber (in mg/100 ml) von an    Gallemangel leidenden Ratten nach fünftägiger Behandlung

|  | P l a s m a | | L e b e r |
|  | Totallipid | Triglycerid | Triglycerid |
|---|---|---|---|
| Kontrolle: | | | |
| vor der Ligatur | 281,  $\pm$  54,5 | 35,4 $\pm$  4,8 | 7,3 $\pm$ 1,6 |
| nach Ligatur | 319,9 $\pm$  32,3 | 28,1 $\pm$  4,3 | 4,2 $\pm$ 0,3 |
| behandelt | | | |
| mit Fett | 528,3 $\pm$ 111,2 | 40,1 $\pm$ 11,5 | 6,4 $\pm$ 1,9 |
| Fett + DIMEB | 688,7 $\pm$  95,8 | 71,9 $\pm$ 19,1 | 9,1 $\pm$ 2,2 |

Tabelle 9

| Die Wirkung einer über 31 Tage fortgesetzten Behandlung mit täglich 150 beziehungsweise 300 mg/kg DIMEB auf normale Ratten (Konz. in mg/100 ml) | | |
|---|---|---|
|  | Triglycerid | |
|  | Plasma | Leber |
| Kontrolle | 52,5 ± 7,4 | 4,5 ± 0,9 |
| 150 mg/kg DIMEB | 48,3 ± 4,5 | 3,6 ± 1,0 |
| 300 mg/kg DIMEB | 45,9 ± 3,1 | 3,8 ± 1,4 |

Beispiel 9

Untersuchung der Absorption von $^3$H-Stearinsäure bei gleichzeitiger Verabreichung von DIMEB an intakten Ratten

Die Wirkung von DIMEB auf die Absorption von $^3$H-Stearinsäure wurde an intakten Ratten intersucht. Ziel des Versuches war es festzustellen, ob die Absorption der Stearinsäure bei physiologischer Gallenfunktion stimuliert wird. Die Nacht vor dem Versuch und für die Dauer des Versuches wurden die Tiere nicht gefüttert, erhielten jedoch Wasser ad libitum. Eine Gruppe (3 Tiere) der durchschnittlich 180 g schweren weiblichen CFY-Ratten erhielt 13 mg $^3$H-Stearinsäure (spezifische Radioaktivität 147,22 kBq/mg), die andere Gruppe (3 Tiere) 11,3 mg $^3$H-Stearinsäure und die Lösung von 50 mg DIMEB in 2 ml destilliertem Wasser. Die Substanzen wurden durch Ultraschall im Wasser suspendiert. Die Radioaktivität des Blures wurde mittels der Methode der Flüssigkeitsszintillation zu unterschiedlichen Zeitpunkten bestimmt (Tabelle 10).

Zwischen der Radioaktivität im Blut der beiden Gruppen konnte kein Unterschied gefunden werden. Eine Stunde nach der Behandlung waren in Blut 1,8-1,9 % der ingesamt eingebrachten Radioaktivität vorhanden, und dieser Wert stieg langsam an. In der 24. Stunde waren 2,7 % der applizierten Radicoaktivität im Blut. Demnach stimuliert bei physiologischer Gallenfunktion DIMEB die Absorption nicht.

Beispiel 10

Untersuchung der Absorption von $^3$H-Stearinsäure bei gleichzeitiger oraler Verabreichung von DIMEB an an Gallenmangel leidenden Ratten

An Ratten, deren Gallenleiter abgebunden war, wurde studiert, wie DIMEB die Galle ersetzt.

Die Tiere - durchschnittlich 180 g schwere weibliche Retten - wurden nach 24 Stunden Futterentzug in Nembutal®-Narkose operiert, ihr Gallenleiter wurde abgebunden. Nach 48 Stunden wurde der Versuch begonnen; zuvor hatten die Tiere 24 Stunden lang kein Futter erhalten. Auch während des Versuches wurde nicht gefüttert, Wasser stand ad libitum zur Verfügung. Die eine Gruppe der Tiere (3 Tiere) erhielt 12,8 mg $^3$H-Stearinsäure (spezifische Radioaktivität 171,12 kBq/mg), die andere Gruppe (3 Tiere) 14,7 mg $^3$H-Stearinsäure und 50 mg DIMEB in 2 ml destilliertem Wasser. Die Substanzen wurden mit Ultraschall im Wasser suspendiert. Die Radioaktivität des Blutes wurde mittels der Methode der Flüssigkeitsszintillation zu unterschiedlichen Zeitpunkten gemessen (Tabelle 11).

Zwischen der Radioaktivität im Blut der beiden Gruppen bestand ein wesentlicher Unterschied. Bei der mit $^3$H-Stearinsäure und DIMEB behandelten Gruppe wurden eine Stunde nach Beginn in 10 ml Blut 1,97 % der insgesamt eingeführten Radioaktivität gemessen, bei der nicht mit DIMEB behandelten Gruppe waren es nur 0,73 %. In der 24. Stunde nach der Verabreichung war der Blutspiegel der auch mit DIMEB behandelten Tiere 1,4 mal so hoch wie der der nur mit Stearinsäure behandelten Tiere. Die Absorption der Fettsäure wurde bei den an Gallenmangel leidenden Tieren demnach durch DIMEB gefördert.

Beispiel 11

Untersuchung der Absorption von $^3$H-Stearinsäure bei gleichzeitiger oraler Verabreichung von DIMEB (an Gallenmangel leidende Ratten)

An Ratten, deren Gallenleiter abgebunden war, wurde studiert, wie DIMEB die Galle ersetzt. Die Tiere - durchschnittlich 150 g schwere weibliche CFY-Ratten - wurden nach 24 Stunden Futterentzug unter Nembutal®-Narkose operiert, ihr Gallenleiter wurde abgebunden. Nach 48 Stunden wurde der Versuch besonnen; zuvor hatten die Tiere 24 Stunden lang kein Futter erhalten. Auch während des Versuches wurde nicht gefüttert, Wasser stand ad libitum zur Verfügung. Eine Gruppe der Ratten (4 Tiere) erhielt 22,2 mg $^3$H-Stearinsäure (spezifische Radioaktivität 92,5 kBq/mg), die andere Gruppe (4 Tiere) 23,5 mg $^3$H-Stearinsäure und 60 mg DIMEB in 2 ml destilliertem Wasser. Die Substanzen wurden mit Ultraschall im Wasser suspendiert. Die Radioaktivität des Blutes wurde mittels der Methode der Flüssigkeitsszintillation zu unterschiedlichen Zeitpunkten bestimmt (Tabelle 12).

Zwischen der Radioaktivität in Blut der beiden Gruppen bestand ein wesentlicher Unterschied. Eine Stunde nach der Verabreichung war der Blutspiegel der auch mit DIMEB behandelten Tiere 4,4 mal so hoch wie der der nur mit Stearinsäure behandelten. Dieser Unterschied wurde langsam geringer, und in der 24. Stunde war der Blutspiegel der mit DIMEB behandelten Tiere nur noch doppelt so hoch wie der der nur mit Stearinsäure behandelten. Durch DIMEB wurde demnach an Tieren, in denen experimentell ein Gallenmangel erzeugt worden war, die Absorption der Fettsäure gefördert.

Tabelle 10

| Zeit h | $^3$H-Stearinsäure | | | | $^3$H-Stearinsäure + DIMEB | | | |
|---|---|---|---|---|---|---|---|---|
| | 1 | 2 | 3 | $\bar{x}$ | 4 | 5 | 6 | $\bar{x}$ |
| 1 | 1,92 | 1,97 | 1,47 | 1,79 | 1,71 | 1,74 | 2,23 | 1,89 |
| 2 | 1,95 | 2,10 | 1,93 | 1,99 | 1,84 | 1,83 | 2,00 | 1,91 |
| 3 | 2,15 | 1,97 | 2,03 | 2,05 | 2,03 | 1,75 | 2,13 | 1,97 |
| 4 | 2,12 | 1,98 | 2,11 | 2,07 | 2,12 | 1,71 | 1,99 | 1,94 |
| 5 | 2,11 | 1,89 | 2,12 | 2,04 | 2,10 | 1,76 | 2,00 | 1,95 |
| 6 | 2,08 | 1,87 | 2,15 | 2,03 | 2,16 | 1,75 | 2,04 | 1,98 |
| 8 | 2,35 | 1,93 | 2,23 | 2,17 | 2,16 | 1,84 | 1,94 | 1,98 |
| 10 | 2,59 | 2,39 | 2,50 | 2,49 | 2,20 | 2,02 | 2,21 | 2,14 |
| 24 | 2,97 | 2,63 | 2,42 | 2,68 | 2,84 | 2,94 | 2,39 | 2,72 |

In 10 ml des Blutes intakter Ratten gemessene Radioaktivität in Prozent der eingebrachten Gesamtradioaktivität (Behandlung: 13 mg $^3$H-Stearinsäure bzw. 11,3 mg $^3$H-Stearinsäure + 50 mg DIMEB in 2 ml destilliertem Wasser)

EP 0 187 653 B1

Tabelle 11

| Zeit h | $^3$H-Stearinsäure | | | | $^3$H-Stearinsäure + DIMEB | | | |
|---|---|---|---|---|---|---|---|---|
| | 1 | 2 | 3 | $\bar{x}$ | 4 | 5 | 6 | $\bar{x}$ |
| 1 | 0,59 | 0,82 | 0,78 | 0,73 | 0,86 | 3,45 | 1,61 | 1,97 |
| 2 | 0,68 | 1,06 | 1,06 | 0,93 | 1,05 | 3,25 | 2,56 | 2,29 |
| 3 | 0,77 | 1,19 | 1,16 | 1,04 | 1,14 | 3,22 | 2,39 | 2,25 |
| 4 | 0,84 | 1,32 | 1,30 | 1,15 | 1,19 | 3,06 | 2,28 | 2,18 |
| 5 | 0,92 | - | 1,50 | 1,21 | 1,24 | 3,08 | 2,27 | 2,20 |
| 6 | 0,93 | 1,50 | 1,51 | 1,31 | 1,25 | 2,91 | 2,56 | 2,24 |
| 7 | 0,96 | 1,64 | 1,64 | 1,43 | 1,33 | 3,30 | 2,67 | 2,34 |
| 8 | 1,10 | 1,65 | 1,67 | 1,47 | 1,37 | 3,13 | 2,58 | 2,36 |
| 10 | 1,10 | 1,84 | 1,57 | 1,50 | 1,29 | 3,26 | 2,67 | 2,41 |
| 24 | 2,44 | 2,60, | 2,09 | 2,38 | 1,60 | 4,56 | 4,00 | 3,39 |

Die in 10 ml Blut von an Gallenmangel leidenden Ratten gemessene Radioaktivität in Prozent der verabreichten Gesamtradioaktivität (Behandlung: 12,8 mg $^3$H-Stearinsäure bzw. 14,7 mg $^3$H-Stearinsäure und 50 mg DIMEB in 2 ml destilliertem Wasser

Tabelle 12

| Gleiche Versuchseinstellung, jedoch 22,2 mg $^3$H-Stearinsäure bzw. 23,5 mg $^3$H-Stearinsäure + 60 mg DIMEB | | | | | | | | | |
|---|---|---|---|---|---|---|---|---|---|
| Zeit h | $^3$H-Stearinsäure | | | | | $^3$H-Stearinsäure + DIMEB | | | |
| | 1 | 2 | 3 | 4 | $\bar{x}$ | 5 | 6 | 7 | 8 | $\bar{x}$ |
| 1 | 0,26 | 0,36 | 0,22 | 0,24 | 0,27 | 1,18 | 1,41 | 1,66 | 0,50 | 1,19 |
| 2 | 0,43 | 0,57 | 0,29 | 0,35 | 0,41 | 1,46 | 1,52 | 1,52 | 0,64 | 1,28 |
| 3 | 0,53 | 0,75 | 0,37 | 0,33 | 0,49 | 1,29 | 1,63 | 1,37 | 0,61 | 1,22 |
| 4 | 0,62 | 0,76 | 0,43 | 0,38 | 0,55 | 1,42 | 1,67 | 1,56 | 0,60 | 1,31 |
| 5 | 0,57 | 0,85 | 0,50 | 0,39 | 0,56 | 1,39 | 1,73 | 1,54 | 0,69 | 1,34 |
| 6 | 0,59 | 0,85 | 0,55 | 0,40 | 0,60 | 1,41 | 1,92 | 1,76 | 0,73 | 1,45 |
| 8 | 0,57 | 0,96 | 0,83 | 0,43 | 0,70 | 1,43 | 1,90 | 1,85 | 0,72 | 1,47 |
| 10 | 0,71 | 0,91 | 0,83 | 0,47 | 0,73 | 1,57 | 1,56 | 1,45 | 0,89 | 1,37 |
| 24 | 0,74 | 0,82 | 0,86 | 1,03 | 0,86 | 1,50 | 1,94 | 1,59 | 1,95 | 1,74 |

**Patentansprüche**

1. Partiell methyliertes $\beta$-Cyclodextrin zur Anwendung als galleersetzenden und die Fettverdauung fördernden Wirkstoff.

2. Heptakis-(2,6-di-0-methyl)-$\beta$-cyclodextrin zur Anwendung als galleersetzenden und die Fettverdauung fördernden Wirkstoff.

3. Arzneimittel, enthaltend partiell methyliertes $\beta$-Cyclodextrin, als galleersetzenden und die Fettverdauung fördernden Wirkstoff, und die üblichen Hilfs- und/oder Trägerstoffe.

4. Arzneimittel nach Anspruch 3, enthaltend Heptakis-(2,6-di-0-methyl)-$\beta$-cyclodextrin als partiell methyliertes $\beta$-Cyclodextrin.

5. Arzneimittel nach Ansprüchen 3 oder 4, enthaltend 5 - 95 %. partiell methyliertes $\beta$-Cyclodextrin und 95 - 5 % der üblichen Hilfs- und/oder Trägerstoffe.

**Claims**

1. Partially methylated $\beta$-cyclodextrin for use as an active substance which replaces bile and promotes the digestion of fat.

2. Heptakis-(2,6-di-0-methyl)-$\beta$-cyclodextrin for use as an active substance which replaces bile and promotes the digestion of fat.

3. Pharmaceutical compositions containing partially methylated $\beta$-cyclodextrin as an active substance which replaces bile and promotes the digestion of fat, as well as conventional excipients and/or carriers.

4. Pharmaceutical compositions according to claim 3, containing heptakis-(2,6-di-0-methyl)-$\beta$-cyclodextrin as the partially methylated $\beta$-cyclodextrin.

5. Pharmaceutical compositions according to claim 3 or 4, containing 5 to 95% of partially methylated $\beta$-cyclodextrin and 95 to 5% of the conventional excipients and/or carriers.

**Revendications**

1. Bêta-cyclodextrine partiellement méthylée pour une utilisation comme substance active remplaçant la bile et favorisant la digestion des graisses.

2. Heptakis-(2,6-di-0-méthyl)-bêta-cyclodextrine pour une utilisation comme substance active remplaçant la bile et favorisant la digestion des graisses.

3. Médicament, contenant de la bêta-cyclodextrine partiellement méthylée en tant que substance active remplaçant la bile et favorisant la digestion des graisses, et les adjuvants et/ou supports classiques.

4. Médicament selon la revendication 3, contenant de l'heptakis-(2,6-di-0-méthyl)-bêta-cyclodextrine comme bêta-cyclodextrine partiellement méthylée.

5. Médicament selon l'une des revendications 3 ou 4, contenant 5 - 95% de bêta-cyclodextrine partiellement méthylée et 95 - 5% des adjuvants et/ou supports classiques.